# EUROPÄISCHE PATENTSCHRIFT

(11) **EP 0 306 873 B1**
(45) Veröffentlichungstag und Bekanntmachung des Hinweises auf die Patenterteilung: **10.02.1993**
(21) Anmeldenummer: 88114430.7
(22) Anmeldetag: 03.09.1988
(51) Int. Cl.: C07C 45/48, C07C 49/395

(54) **Verfahren zur Herstellung von Cyclopentanon**
Process for the preparation of cyclopentanone
Procédé pour la préparation de la cyclopentanone

(30) Priorität: 09.09.1987 DE 3730185
(43) Veröffentlichungstag der Anmeldung: 15.03.1989
(73) Patentinhaber: BASF Aktiengesellschaft, 67063 Ludwigshafen (DE)
(72) Erfinder: Decker, Martin, Dr., D-6700 Ludwigshafen (DE); Wache, Harro, Dr., D-6701 Fussgoenheim (DE); Franzischka, Wolfgang, Dr., D-6710 Frankenthal (DE)

(56) Entgegenhaltungen:
- EP-A- 0 093 853
- DE-B- 1 768 138
- FR-A- 1 270 909
- US-A- 2 612 524

## Beschreibung

Die Erfindung betrifft das in den Ansprüchen angegebene Verfahren.

Aus der FR-A-12 70 909 ist ein Verfahren zur Herstellung von Säureanhydriden und/oder Ketonen durch Erhitzen gesättigter aliphatischer Monocarbonsäuren mit mindestens 3 C-Atomen im Molekül, in Gegenwart von Katalysatoren bekannt, wobei man diese Säuren gasförmig durch ein auf 250 bis 800°C erhitztes Katalysatorfließbett, bestehend aus auf einer Trägersubstanz aufgebrachten Oxyden, Hydroxyden oder Phosphaten von Elementen der II. bis IV. Hauptgruppe oder der III. bis VIII. Nebengruppe des Periodensystems, hindurchleitet und aus den abziehenden Gasen durch Kühlung die Umsetzungsprodukte gewinnt.

Die Herstellung von Cyclopentanon aus Adipinsäure ist bekannt. In der DE-A 256 622 ist die Umsetzung von geschmolzener Adipinsäure in Gegenwart von Metalloxiden oder Metallpulvern zu Cyclopentanon beschrieben. Bei dieser Arbeitsweise stellt die korrosive Wirkung des Reaktionsgemisches ein großes Problem dar. Ferner entstehen außer den cyclischen Ketonen lineare Polyketodicarbonsäuren, s. z.B. Chem. Abstr. Vol. 80, (1974), 47408 f und Chem. Abstr. Vol. 98, (1983), 34199 k.

Als Nebenausbeute entsteht Cyclopentanon in kleiner Menge und unreiner Form auch bei der Umsetzung von Adipinsäure mit Ammoniak zu Adipodinitril, bei dessen Destillation es aus den Vorläufen gewonnen werden kann, s. DE-A 743 967. Die Reinigung dieses Produktes bereitet jedoch große Schwierigkeiten.

Beim erfindungsgemäßen Verfahren werden die genannten Nachteile vermieden und Cyclopentanon wird in hoher Ausbeute und Reinheit erhalten. Die bei der Umsetzung der Dicarbonsäure verwendeten Katalysatoren werden vorteilhaft in feiner Körnung als Wirbelschicht eingesetzt und die Dicarbonsäure als festes Pulver mit dem Inertgas, vorzugsweise Stickstoff, in die heiße Katalysatorwirbelschicht eingeblasen.

Als Katalysatorträger eignet sich z.B. Kieselsäure in einer Körnung von 0,05 bis 0,3 mm, die die katalytisch aktiven Substanzen enthält. Die Kieselsäure enthält Phosphorsäure oder das Mono-Natriumsalz der Phosphorsäure als aktive Substanz. Der Katalysator enthält die Phosphorverbindungen z.B. in Mengen bis zu 10 % und kann z.B. durch Mischen von Kieselsäuregel und Phosphorsalz, Versprühen und Kalzinieren der Mischung hergestellt werden. Bei der erfindungsgemäßen Umsetzung erhält man mit diesem Katalysator Cyclopentanon in hoher Ausbeute und guter Reinheit.

Ausbeute und Reinheit des Cyclopentanons werden verbessert, wenn man je Mol Dicarbonsäure dem Trägergas noch 2 bis 10, vorzugsweise 4 bis 6 Mol Wasserdampf beifügt.

Bei den sauren Katalysatoren ist eine Wasserzugabe nicht erforderlich.

Die Umsetzung der Dicarbonsäuren erfolgt in der Gasphase bei Temperaturen von 200 bis 450°C, vorzugsweise bei 300 bis 400°C. Mit den sauren Katalysatoren wird der obere Bereich dieser Temperaturspanne bevorzugt. Im allgemeinen wird die Reaktion unter Normaldruck oder schwach erhöhtem Druck bis zu etwa 2 bar durchgeführt. Es ist jedoch auch möglich, die Reaktion unterhalb des Normaldrucks durchzuführen, was zur besseren Verdampfung der Dicarbonsäure vorteilhaft sein kann.

Besonders vorteilhaft ist es, daß nach dem erfindungsgemäßen Verfahren Korrosionen an den Produktionsanlagen vermieden und das Reaktionsprodukt in hoher Ausbeute und Reinheit erhalten wird. Cyclopentanon ist ein wertvolles Zwischenprodukt, das hauptsächlich für Wirkstoffsynthesen verwendet wird.

### Beispiel 1

In einen Wirbelschichtreaktor mit 60 mm Durchmesser werden 355 g Katalysator mit einer Körnung von 0,06 bis 0,30 mm eingefüllt. Der Katalysator besteht aus einer Mischung von 90 % Kieselsäure und 10 % Natrium-dihydrogenphosphat.

Durch einen Verteilerboden werden je Stunde 200 Nl Stickstoff von 360°C in den Reaktor geleitet und der Katalysator in wirbelnde Bewegung versetzt. Dem Stickstoff werden 163 g Wasser je Stunde zugegeben, die zusammen mit dem Stickstoff dampfförmig in die Katalysatorschicht gelangen.

Unmittelbar oberhalb des Verteilerbodens befindet sich am Reaktor ein seitlicher Stutzen, durch den je Stunde 200 g feste Adipinsäure mit 200 Nl/h Stickstoff von ca. 20°C in die Katalysatorschicht eingeblasen werden. Die Katalysatorschicht wird mit Hilfe einer elektrischen Heizung auf 400°C eingeregelt. Die Reaktionsgase verlassen den Reaktor über ein Filter und werden in einem Waschturm durch Umpumpen von Methanol abgekühlt und kondensiert.

Insgesamt werden 1 810 g Adipinsäure umgesetzt und 3 083 g Austrag mit 909,3 g Cyclopentanon erhalten. Die Ausbeute beträgt 87,3 Mol. %.

### Beispiel 2

Auf gleiche Weise wie in Beispiel 1 werden dem Reaktor je Stunde 214 g Adipinsäure zugeführt, jedoch wird die Zufuhr von Wasser zum Wirbelgasaufheizer unterlassen. Im übrigen werden die Reaktionsbedingungen und die Kondensation der Reaktionsprodukte wie im Beispiel 1 beibehalten.

Es werden dem Reaktor insgesamt 2 570 g Adipinsäure zugeführt und eine Lösung der Reaktionsprodukte in Methanol von 2 593 g erhalten. Die Lösung enthält 1 307,1 g Cyclopentanon, was einer Ausbeute von 87,6 Mol.% entspricht.

## Patentansprüche

1. Verfahren zur Herstellung von Cyclopentanon durch Umsetzung von Adipinsäure unter Inertgasatmosphäre in der Gasphase bei 200 bis 450°C gegebenenfalls in Gegenwart von Wasserdampf, dadurch gekennzeichnet, daß man Kieselsäure als oxidisch sauren Katalysatorträger und Phosphorsäure oder das Mononatriumsalz der Phosphorsäure als aktive Substanz verwendet.

2. Verfahren nach Anspruch 1, dadurch gekennzeichnet, daß man dem Inertgas, bezogen auf die Adipinsäure, 2 bis 10 Mol Wasserdampf zugibt.

3. Verfahren nach Anspruch 1, dadurch gekennzeichnet, daß man dem Inertgas, bezogen auf die Adipinsäure, 4 bis 6 Mol Wasserdampf zugibt.

4. Verfahren nach Anspruch 1, dadurch gekennzeichnet, daß man als Inertgas Stickstoff verwendet.

5. Verfahren nach Anspruch 1, dadurch gekennzeichnet, daß man das Verfahren in der Wirbelschicht durchführt.

6. Verfahren nach Anspruch 1, dadurch gekennzeichnet, daß man die Adipinsäure in fester Form in die Wirbelschicht einbringt.

## Claims

1. A process for the preparation of cyclopentanone by converting adipic acid in the gas phase at from 200 to 450°C under an inert gas atmosphere, in the presence or absence of steam, which comprises using silicic acid as oxidically acidic catalyst carrier and phosphoric acid or the monosodium salt of phosphoric acid as active substance.

2. A process as claimed in claim 1, wherein from 2 to 10 mol, based on the adipic acid, of steam are added to the inert gas.

3. A process as claimed in claim 1, wherein from 4 to 6 mol, based on the adipic acid, of steam are added to the inert gas.

4. A process as claimed in claim 1, wherein the inert gas used is nitrogen.

5. A process as claimed in claim 1, wherein the process is carried out in a fluidized bed.

6. A process as claimed in claim 1, wherein the adipic acid is introduced into the fluidized bed in solid form.

## Revendications

1. Procédé de préparation de la cyclopentanone par la réaction de l'acide adipique sous une atmosphère d'un gaz inerte, en phase gazeuse, à 200-450°C, éventuellement en présence de vapeur d'eau, caractérisé en ce que l'on utilise de l'acide silicique à titre de support de catalyseur acide oxydique et de l'acide phosphorique ou le sel monosodique de l'acide phosphorique à titre de substance active.

2. Procédé suivant la revendication 1, caractérisé en ce que l'on ajoute de 2 à 10 moles de vapeur d'eau au gaz inerte, par rapport à l'acide adipique.

3. Procédé suivant la revendication 1, caractérisé en, ce que l'on ajoute de 4 à 6 moles de vapeur d'eau au gaz inerte, par rapport à l'acide adipique.

4. Procédé suivant la revendication 1, caractérisé en ce que l'on utilise de l'azote à titre de gaz inerte.

5. Procédé suivant la revendication 1, caractérisé en ce qu'on le met en oeuvre en lit fluidisé.

6. Procédé suivant la revendication 1, caractérisé en ce que l'on introduit l'acide adipique sous forme solide dans le lit fluidisé.
